# EUROPÄISCHE PATENTSCHRIFT

(11) **EP 1 226 815 B1**
(45) Veröffentlichungstag und Bekanntmachung des Hinweises auf die Patenterteilung: **23.07.2003**
(21) Anmeldenummer: 02001660.6
(22) Anmeldetag: 24.01.2002
(51) Int. Cl.: A61K 7/09

(54) **Mittel zur dauerhaften Verformung von menschlichen Haaren**
Composition for the permanent deformation of hair
Composition pour la déformation permanente des cheveux

(30) Priorität: 26.01.2001 DE 10103494
(43) Veröffentlichungstag der Anmeldung: 31.07.2002
(73) Patentinhaber: KPSS-Kao Professional Salon Services GmbH, 64297 Darmstadt (DE)
(72) Erfinder: Wood, Jonathan, Dr., 69469 Weinheim (DE); Rose, Burkhard, 64297 Darmstadt (DE)

(56) Entgegenhaltungen:
- EP-A- 0 363 057
- EP-A- 0 614 657
- EP-A- 0 666 073
- US-A- 5 866 104
- US-A- 6 113 888

## Beschreibung

Die vorliegende Erfindung betrifft ein Mittel zur dauerhaften Verformung von menschlichen Haaren, und zwar sowohl ein Dauerwellmittel mit guter Verformungswirksamkeit, das jedoch gleichwohl eine haarschonende Behandlung gewährleistet, als auch ein Entkräuselungsmittel, d.h., ein Mittel zum Entkräuseln (Glätten) von gewelltem Haar.

Die Dauerwellung erfolgt bekanntlich in zwei Behandlungsschritten, der reduktiven Spaltung der Cystin-Disulfidbrücken des Haares durch Einwirkung eines Reduktionsmittels, und die anschließende Neutralisierung bzw. Fixierung durch Aufbringung eines Oxidationsmittels, wodurch die Cystin-Disulfidbrücken wiederhergestellt werden.

Das überwiegend eingesetzte Reduktionsmittel ist auch heute noch die Thioglykolsäure, auch in Form ihrer Salze, insbesondere des Ammoniumsalzes, obwohl zahlreiche andere Thioverbindungen für diesen Zweck vorgeschlagen wurden, die sich jedoch in der Praxis zumeist nicht durchgesetzt haben.

Die Thioglykolat enthaltenden Zusammensetzungen werden üblicherweise bei einem pH-Wert zwischen etwa 7 und 10, insbesondere 8,5 und 9,5, eingesetzt.

Die vorliegende Erfindung geht von der Aufgabenstellung aus, ein Mittel zum dauerhaften Verformen von menschlichen Haaren zu schaffen, das einerseits eine gute Verformungswirksamkeit aufweist, andererseits jedoch die Haarschädigung verringert und insbesondere als Dauerwellmittel oder als Mittel zum Entkräuseln, d.h. Glätten, von Kraushaar verwendet werden kann.

Die Lösung dieser Aufgabe besteht darin, einem Verformungsmittel auf wäßriger Basis, enthaltend mindestens ein Reduktionsmittel und 2-Methyl-1,3-propandiol in einer Menge zwischen 0,1 und 15 Gew.-%, vorzugsweise 0,25 bis 10 Gew.-%, insbesondere 0,5 bis 5 Gew.-%, berechnet auf die Gesamtzusammensetzung, zuzusetzen.

Die Verwendung der verschiedensten Polyole als Lösungsmittel sowie Lösungs- und Penetrationsvermittler in Dauerwellmitteln ist bereits seit langem bekannt. So beschreibt die US-A 3, 433, 868 bereits zweiphasige Dauerwellzusammensetzungen mit schnellbrechendem Schaum, die zusätzlich Polyalkohole enthalten können. Als solche sind Glycerein, 1,3-Butylenglykol, Propylenglykol, 1,2,6-Hexan-triol, 1,5-Pentandiol, 2-Methylpentandiol-2,4, 2-Ethylhexandiol-1,3 und verschiedene Oligound Polydiole genannt.
Die EP 0 363 057 B1 betrifft die Verwendung von 1,3-Alkyldiolen und 1,3-Alkandiolen in Dauerwellmitteln, vorzugsweise 2-Ethyl-1,3-hexandiol und 1,3-Butandiol.

Durch diese Verbindungen wird jedoch kein zufriedenstellender Effekt erzielt; mehrere von ihnen wie Butandiole können auch Hautirritationen oder -sensibilisierungen hervorrufen.

Die erfindungsgemäßen Dauerwellmittel enthalten vorzugsweise eine reduzierende Thioverbindung. Bevorzugt sind Thioglykolsäure und Thiomilchsäure sowie deren Salze, insbesondere die Ammonium- und Ethanolaminsalze.
Weitere einsetzbare Thioverbindungen sind insbesondere Cystein bzw. dessen Hydrochlorid, Homocystein, Cysteamin, N-Acetylcystein, Thioglycerin, Ethandiolmonothioglykolat, 1,2-Propylenglykolmonoglykolat (vgl. auch WO-A 93/1791), 1,3-Propandiolmonothioglykolat bzw. das daraus resultierende Isomerengemisch, 1,3-Butandiol- und 1,4-Butandiolmonothioglykolat bzw. deren Isomerengemische, Polyethylenglykol- wie Di-, Tri- und Tetraethylenglykolmonothioglykolate, Glycerinmonothiolactat und weitere Thiosäuren und deren Ester sowie Gemische derselben.

Auch die Verwendung anorganischer reduzierender Schwefelverbindungen wie Natriumhydrogensulfit ist prinzipiell möglich.
Der Gesamtgehalt an Reduktionsmitteln in den erfindungsgemäßen Zusammensetzungen beträgt üblicherweise 2,5 bis etwa 15 Gew.-%, berechnet auf freie Thioglykolsäure als Bezugssubstanz.

Die Reduktionsmittel enthaltenden Dauerwellpräparate können, falls erforderlich, einen Gehalt an Alkalisierungsmitteln aufweisen. Die Menge ist abhängig vom reduzierenden Wirkstoff und dem angestrebten pH-Wert der Zusammensetzung. Vorzugsweise enthält die Reduktionsmittel-Zusammensetzung etwa 0,1 bis etwa 5, insbesondere etwa 0,5 bis etwa 2,5 Gew.-% desselben.
Bevorzugte Alkalisierungsmittel im Rahmen der Erfindung sind Ammoniumcarbamat, Ammoniak und/oder Ammonium(bi)carbonat. Es wird die Einstellung eines pH-Wertes im Bereich zwischen etwa 6,5 und etwa 9,5, vorzugsweise etwa 7 bis 8,5, angestrebt.

Die erfindungsgemäßen Verformungsmittel sind sowohl zur Dauerwellung, also Lockung des menschlichen Haares, als auch zur Entkräuselung, also Glättung desselben, verwendbar.

Es hat sich dabei gezeigt, daß für die erfindungsgemäßen Dauerwellmittel eine Viskosität von etwa 500 bis 10 000, vorzugsweise etwa 1000 bis etwa 5000 mPa.s, gemessen bei 20°C im Brookfield-Viskosimeter (Spindel Nr.5), besonders geeignet ist, während sich für Entkräuselungsmittel vorzugsweise eine höhere Viskosität bis zu etwa 50 000 mPa.s, gemessen bei 20°C im Brookfield-Viskosimeter (Spindel Nr.5), eignet.
Die Viskositätseinstellung erfolgt jeweils durch Zusatz entsprechender Mengen von an sich bekannten Verdickungsmitteln wie Cellulosederivaten, Polyelektrolyten, etc, oder vorzugsweise durch den Zusatz von C₁₀-C₂₂-Fettalkoholen, insbesondere im Gemisch mit langkettigen quatemären Ammoniumverbindungen, in einer bevorzugten Menge von 1 bis 15 Gew.-%, insbesondere 2 bis 10 Gew.-% Fettalkohol.

Die erfindungsgemäß zum Einsatz kommenden Dauerwellmittel enthalten vorzugsweise auch Tenside. Deren Anteil liegt bei etwa 0,1 bis etwa 10, insbesondere etwa 1 bis etwa 5 Gew.-% der das Reduktionsmittel enthaltenden Zusammensetzung.

Sowohl bei den in den Reduktionsmittel-Zusammensetzungen als auch bei den in den Fixiermitteln eingesetzten Tensiden handelt es sich vorzugsweise um die bekannten Produkte, die gegebenenfalls auch in Kombination untereinander zum Einsatz gelangen.

Geeignete anionische Tenside sind insbesondere die bekannten Alkylethersulfate und -carbonsäuren, insbesondere in Form ihrer Alkalisalze, sowie Eiweiß-Fettsäure-Kondensate.

Geeignete nichtionische Tenside, die im Rahmen der Erfindung bevorzugt sind, sind insbesondere C₈-C₁₈-Fettalkoholpolyglykolether, Fettsäurepolyglykolester, Fettsäurealkanolamide, Aminoxide und vor allem C₈-C₁₈-Alkylpolyglucoside.

Es können auch amphotere Tenside wie die bekannten Betaine und Amidobetaine sowie, gemäß einer weiteren bevorzugten Ausführungsform, kationaktive Tenside wie quatemäre Ammoniumverbindungen, insbesondere in einer Menge von 0,05 bis 5, vor allem 0,1 bis 2,5 Gew.-%, berechnet auf die reduktionsmittelhaltige Zusammensetzung, eingesetzt werden.
Geeignete langkettige quatemäre Ammoniumverbindungen, die allein oder im Gemisch miteinander eingesetzt werden können, sind insbesondere Cetyltrimethylammoniumchlorid, Dimethyldicetylammoniumchlorid, Trimethylcetylammoniumchlorid, Stearyltrimethylammoniumchlorid, Dimethylstearylbenzylammoniumchlorid, Benzyltetradecyldimethylammoniumchlorid, Dimethyldihydriertes-Talgammoniumchlorid, Laurylpyridiniumchlorid, Lauryldimethylbenzylammoniumchlorid, Lauryltrimethylammoniumchlorid, Tri(oligooxyethyl)alkylammoniumphosphat, Cetylpyridiniumchlorid, etc.
Gut geeignet sind auch die in der EP-A 472 107 geoffenbarten quaternären Ammoniumsalze.

Im Prinzip sind alle quatemären Ammoniumverbindungen, wie sie im "CTFA International Cosmetic Ingredient Dictionary", 4th Ed. (1991), unter dem Trivialnamen "Quaternium" aufgeführt sind, geeignet.

Ein weiterer wünschenswerter Bestandteil der erfindungsgemäß verwendeten Reduktionsmittel-Zusammensetzungen sind, in Addition zu 2-Methyl-1,3-propandiol, weitere Mono- und Dialkohole bzw. deren Ether, insbesondere Mono-C₁-C₃alkylether.

Bevorzugte Substanzen sind in diesem Zusammenhang Ethanol, n-Propanol, Isopropylalkohol, 1-Methoxypropanol(-2), 1-Ethoxypropanol(-2) und Ethoxydiglykol, vorzugsweise in einer Menge von etwa 0,1 bis 10 Gew.-%, berechnet auf die Gesamtzusammensetzung.
Auch weitere Diole und deren Ether können in untergeordneten Mengen mitverwendet werden, z.B. 1,3- und 1,4-Butandiol, Diethylenglykol und dessen Monomethyl- und Monoethylether sowie Dipropylenglykol und dessen Monomethylund Monoethylether.
Auch Glycerin, Hexantriol, Ethylcarbitol, Benzylalkohol, Benzyloxyethanol sowie Propylencarbonat (4-Methyl-1,3-dioxolan-2-on), N-Alkylpyrrolidone und Harnstoff können Verwendung finden.

Weitere mögliche zusätzliche Bestandteile sind kationische, anionische, nichtionische und amphotere Polymere, vorzugsweise in einer Menge von etwa 0,1 bis etwa 5, insbesondere etwa 0,25 bis 2,5 Gew.-% der Gesamtzusammensetzung des Mittels.

Die erfindungsgemäß eingesetzten Mittel können selbstverständlich alle in dauerhaften Verformungsmitteln üblichen Stoffe enthalten, auf deren detaillierte Aufzählung hier verzichtet wird, und liegen vorzugsweise als Lösungen, mehr oder weniger viskose Gele, Emulsionen bzw. Cremes vor.

Es kann sich dabei um einphasige Produkte oder um in getrennten Verpackungen untergebrachte Zusammensetzungen handeln, die bei der Anwendung vereinigt werden, wie sie z.B. in der DE-C 43 04 828 beschrieben sind.

Zur Vermeidung von Wiederholungen wird hierzu auf den Stand der Technik verwiesen, wie er beispielsweise in "Ullmanns's Encyclopedia of Industrial Chemistry", Vol. A12 (1986), S. 588 bis 591, sowie insbesondere in der Monographie von K. Schrader, "Grundlagen und Rezepturen der Kosmetika", 2. Auflage (1989, Hüthig-Verlag), S. 823 bis 840, sowie in dem Übersichtsartikel von D. Hollenberg et. al. in "Seifen-Öle-Fette-Wachse", **117** (1991), S. 81-87, beschrieben ist.

Die dort geoffenbarten Zusammensetzungen und Einzelbestandteile, auf die ausdrücklich Bezug genommen wird, können auch im Rahmen der vorliegenden Erfindung verwendet werden.

Falls erwünscht, kann vor dem Auftrag des Reduktionsmittels noch ein Vorbehandlungsmittel appliziert werden, wie es beispielsweise in der DE-A 37 40 926 beschrieben ist. Nach dem Aufbringen dieses Vorbehandlungsmittels wird das Haar aufgewickelt und die Reduktionsmittel-Zusammensetzung aufgetragen. Nach etwa 15- bis 30-minütiger Einwirkung und Spülung erfolgt die Fixierung mit üblichen, aus dem Stand der Technik hinreichend bekannten Peroxid- oder Bromat-Zusammensetzungen.
Ebenso kann selbstverständlich auch eine an sich bekannte Zwischenbehandlung zwischen Reduktions- und Neutralisierungsphase erfolgen.

Die folgenden Beispiele dienen der Illustration der Erfindung.

### Beispiel 1:

| **Alkalische Dauerwelle für normales Haar** | |
|---|---|
| Ammoniumthioglykolat (60%-ig) | 21,3 (Gew.-% |
| Ammoniumhydrogencarbonat | 5,0 |
| 2-Methyl-1,3-propandiol | 3,0 |
| PEG-40-Hydriertes Ricinusöl | 0,7 |
| Polyquatemium-35 | 0,5 |
| Parfum | 0,4 |
| Ammoniak, 25%-ig | ad pH 8,3 |
| Wasser | ad 100,0 |

Mit dieser Zusammensetzung wurde in üblicher Weise etwa 15 Minuten dauergewellt, gespült und mit einer üblichen 2,5%-igep H₂O₂-Zusammensetzung etwa 8 Minuten fixiert.
Es wurde eine betonte, formschöne Dauerwellung erhalten.
Ersatz des 2-Methyl-1,3-propandiols durch die gleiche Menge 1,3-Butylenglykol führte zu einem wesentlich konturenschwächer ausgebildeten Wellbild.

### Beispiel 2:

| **Alkalische Dauerwelle für geschädigtes Haar** | |
|---|---|
| Ammoniumthioglykolat (60%-ig) | 15,0 (Gew.-%) |
| Ammoniumhydrogencarbonat | 2,5 |
| Ceteth-20 | 0,7 |
| Tallowtrimoniumchlorid | 0,1 |
| 2-Methyl-1,3-propandiol | 0,5 |
| Polyquaternium-6 | 1,5 |
| Parfum | 0,4 |
| Ammoniak, 25%-ig | ad pH 8,0 |
| Wasser | ad 100,0 |

Mit dieser Zusammensetzung wurde eine gleichartige Dauerwellung wie mit jener nach Beispiel 1 erhalten.

Ersatz des 2-Methyl-1,3-propandiols durch 2-Methyl-2,4-pentandiol führte zu einem konturenschächeren Wellbild.

### Beispiel 3:

### Neutraldauerwelle für normales Haar

Ein aus zwei Zusammensetzungen A und B bestehendes Dauerwellmittel, abgepackt in zwei bis zur Anwendung getrennt gehaltenen Abteilungen einer Zweikammerdose, wurde vor der Anwendung durch Durchstoßen der Trennwand hergestellt und auf gewickeltes menschliches Haar aufgebracht, nach etwa fünfzehnminütiger Einwirkung gespült und mit einer 2,5%-H₂O₂-Fixierzusammensetzung etwa fünf Minuten fixiert, erneut gespült, gewaschen und getrocknet.
Es wurde eine ausdrucksvolle, gleichmäßige, intensive Dauerwelle erhalten.
Eine identische Behandlung, wobei jedoch anstelle von 0,5 Gew.-% 2-Methylpropandiol-1,3 die gleiche Menge 2-Ethyl-1,3-hexandiol zugesetzt wurde, war im Wellbild sichtbar unterlegen.

| **Zusammensetzung A**: | |
|---|---|
| Ammoniumhydrogencarbonat | 4,5 (g) |
| Polyquatemium-6 | 1,0 |
| PEG-65-Hydriertes Ricinusöl | 0,8 |
| Isopropylalkohol | 1,5 |
| Ethoxydiglykol | 2,0 |
| Cocoamidopropylbetain | 1,0 |
| Parfum | 0,3 |
| Trübungsmittel | 0,5 |
| Ammoniak, 25%-ig | ad pH 8,4 |
| Wasser | ad 72,0 |

| **Zusammensetzung B:** | |
|---|---|
| Ammoniumthioglykolat, 70%-ig | 18,0 (g) |
| Thiomilchsäure | 2,0 |
| 2-Methyl-1,3-propandiol | 0,5 |
| Ammoniak, 25%-ig | ad pH 5,5 |
| Wasser | ad 28,0 |

Nach dem Vermischen beider Zusammensetzungen wurde ein gebrauchsfertiges Produkt mit einem pH-Wert von 7,4 erhalten.

### Beispiel 4:

### Neutraldauerwelle für gefärbtes Haar

Analog Beispiel 4 wurde ein in eine Zweikammerverpackung abgepacktes Dauerwellprodukt hergestellt:

| **Zusammensetzung A:** | |
|---|---|
| Ammoniumhydrogencarbonat | 3,5 (g) |
| Polyquaternium-11 | 0,5 |
| Ethanol | 0,5 |
| 1-Methoxypropanol (-2) | 1,0 |
| Cocoamidopropylbetain | 1,0 |
| PEG-25-glycerylcocoat | 0,8 |
| Parfum | 0,3 |
| Trübungsmittel | 0,5 |
| Ammoniak, 25%-ig | ad pH 8,3 |
| Wasser | ad 72,0 |

| **Zusammensetzung B:** | |
|---|---|
| Ammoniumthioglykolat, 70%-ig | 13,0 (g) |
| Thiomilchsäure | 0,5 |
| 2-Methyl-1,3-propandiol | 1,5 |
| Ammoniak, 25%-ig | ad pH 5,5 |
| Wasser | ad 28,0 |

Durch Vermischung der Zusammensetzungen unmittelbar vor der Anwendung wurde ein Produkt mit einem pH-Wert von 7,4 erhalten. Diese Mischung ergab nach der im Beispiel 3 beschriebenen Anwendung auf gefärbtem Haar eine ausdrucksvolle Dauerwelle, die den Farbglanz und die Farbintensität in keiner Weise beeinträchtigte.

### Beispiel 5:

| **Alkalisches Dauerwell-Gel** | |
|---|---|
| Ammoniumthioglykolat, 70%-ig | 15,0 (g) |
| Ammoniumhydrogencarbonat | 4,5 |
| PEG-40-Hydriertes Ricinusöl | 0,7 |
| C₁₂-C₁₈-Fattalkoholgemisch | 3,5 |
| 2-Methyl-1,3-propandiol | 0,5 |
| Polyquaternium -28 | 0,1 |
| Parfum | 0,3 |
| Ammoniak, 25%-ig | ad pH 8,0 |
| Wasser | ad 100,0 |

### Beispiel 6:

| **Entkräuselungsmittel** | |
|---|---|
| Thioglykolsäure | 8,0 (Gew.-%) |
| C₁₆-C₂₂-Fettalkoholgemisch | 3,5 |
| Oleth-50 | 2,5 |
| Laureth-23 | 1,5 |
| Polyquaternium-2 | 0,8 |
| 2-Methyl-1,3-propandiol | 5,0 |
| Parfum | 0,6 |
| Monoethanolamin | ad pH 9,0 |
| Wasser | ad 100,0 |

Diese Zusammensetzung stellt ein wirksames Entglättungsmittel für gekräuseltes Haar dar.
Keines der erfindungsgemäßen Dauerwell- bzw. Entkräuselungsmittel nach den Beispielen 1 bis 6 zeigte, auch nach wiederholter Anwendung, die geringste irritierende oder sensibilisierende Wirkung.

## Patentansprüche

1. Mittel zur dauerhaften Verformung von menschlichen Haaren, enthaltend mindestens ein Reduktionsmittel, **dadurch gekennzeichnet, daß** es 0,1 bis 15 Gew.-%, berechnet auf die Gesamtzusammensetzung, 2-Methyl-1,3-propandiol enthält.

2. Mittel nach Anspruch 1, enthaltend 0,25 bis 5 Gew.-%, berechnet auf die Gesamtzusammensetzung, 2-Methyl-1,3-propandiol.

3. Mittel nach Anspruch 1 oder 2, enthaltend 0,1 bis 5 Gew.-%, berechnet auf die Gesamtzusammensetzung, mindestens eines nichtionischen Tensids.

4. Mittel nach einem oder mehreren der Ansprüche 1 bis 3, **dadurch gekennzeichnet, daß** es eine Brookfield-Viskosität von 500 bis 10 000 mPa.s bei 20°C aufweist.

5. Mittel nach einem oder mehreren der Ansprüche 1 bis 4, enthaltend 0,1 bis 10 Gew.-%, berechnet auf die Gesamtzusammensetzung, 1-Methoxypropanol (-2) und/oder Ethoxydiglykol.

## Claims

1. Composition for the permanent shaping of human hair, comprising at least one reducing agent, **characterized in that** it comprises 0.1 % to 15 % by weight, calculated to the total composition, of 2-methyl-1.3-propanediol.

2. Composition according to claim 1, comprising 0.25 % to 5 % by weight, calculated to the total composition, of 2-methyl-1.3 propanediol.

3. Composition according to claim 1 or 2, comprising 0.1 % to 5 % by weight, calculated to the total composition, of at least one nonionic surfactant.

4. Composition according to one or more of claims 1 to 3, **characterized in that** it has a Brookfield viscosity of 500 to 10,000 mPa.s at 20°C.

5. Composition according to one or more of claims 1 to 4, comprising 0.1 % to 10 % by weight, calculated to the total composition, of 1-methoxypropanol (-2) and/or ethoxydiglycol.

## Revendications

1. Agent pour la déformation permanente des cheveux humains, qui contient au moins un agent réducteur, **caractérisé en ce qu'**il contient de 0,1 à 15 % en poids, calculés par rapport à la composition totale, de 2 méthyl-1,3-propanediol.

2. Agent selon la revendication 1, qui contient de 0,25 à 5 % en poids, calculés par rapport à la composition totale, de 2-méthyl-1,3-propanediol.

3. Agent selon la revendication 1 ou 2, qui contient de 0,1 à 5 % en poids, calculés par rapport à la composition totale, d'au moins un agent tensioactif non ionique.

4. Agent selon l'une ou plusieurs des revendications 1 à 3, **caractérisé en ce qu'**il présente une viscosité Brookfield de 500 à 10 000 mPa.s à 20°C.

5. Agent selon l'une ou plusieurs des revendication s 1 à 4, qui contient de 0,1 à 10 % en poids, calculés par rapport à la composition totale, de 1-méthoxypropanol(-2) et/ou d'éthoxydiglycol.
